# EUROPEAN PATENT APPLICATION

(11) **EP 1 982 959 A2**
(43) Date of publication of application: **22.10.2008**
(21) Application number: 08251131.2
(22) Date of filing: 27.03.2008
(51) Int. Cl.: C02F 1/28, C02F 1/42, B01J 41/04, B01J 45/00

(54) **Method for removing phosphate from aqueous solutions**

(30) Priority: 20.04.2007 US 925492 P
(71) Applicant: Rohm and Haas Company, Philadelphia, PA 19106-2399 (US)
(72) Inventor: Black, Richard, Bally, Pennsylvania 19503 (US); Hughes, Lyn, Harylesville, Pennsylvania 19438 (US); Trejo, Jose Antonio, Lansdale, Pennsylvania 19446 (US)
(74) Representative: Kent, Venetia Katherine

(57) **Abstract**

A method for removing phosphate ion from an aqueous solution containing phosphate ion using a resin loaded with a hydrous oxide of an amphoteric metal ion. The resin loaded with a hydrous oxide of an amphoteric metal ion is produced by combining a resin with at least two bed volumes of an aqueous solution containing a salt of the amphoteric metal ion, and having a metal ion concentration of at least 5%, and then treating with an aqueous alkali metal hydroxide solution.

## Description

This invention relates to a method for removing phosphate ion from aqueous solutions using an ion exchange resin loaded with a hydrous oxide of an amphoteric metal.

Hyperphosphatemia is a condition characterized by abnormally high serum phosphate levels. A variety of phosphate-binding polymeric materials has been suggested for treatment of this condition, either by ingestion or by external treatment of body fluids, e.g., hemodialysis. For example, weak base anion exchange resins chelated to ferric ions are reported in U.S. Pat. No. 6,180,094. However, there is a need for additional materials capable of removing phosphate from the gastrointestinal tract. Moreover, this reference discloses incorporation of metals only by complexation to weakly basic anion exchange groups, thereby limiting the metal content of the resin.

The problem addressed by this invention is to provide additional materials useful for removing phosphate ion from aqueous solutions.

### STATEMENT OF THE INVENTION

The present invention is directed to a process for removing phosphate ion from an aqueous solution containing phosphate ion. The method comprises steps of: (a) mixing a resin with at least two bed volumes of an aqueous solution containing a salt of an amphoteric metal ion, and having a metal ion concentration of at least 5%; (b) draining excess liquid from the resin; (c) adding at least 0.3 bed volumes of an aqueous alkali metal hydroxide solution having an alkali metal hydroxide concentration of at least 3%, while monitoring pH, at a rate sufficient to raise liquid-phase pH above 4 within 20 minutes; (d) mixing while adding additional aqueous alkali metal hydroxide solution to maintain liquid-phase pH between 4 and 12; (e) draining excess liquid from the resin; and (f) combining the resin with an aqueous solution containing phosphate ion.

The present invention is further directed to a process for removing phosphate ion from an aqueous solution containing phosphate ion by contacting the aqueous solution with a resin comprising 10% to 50% of an amphoteric metal ion which is present as a hydrous oxide; wherein at least 50% of said metal ion is located in an outer half of a resin bead volume.

### DETAILED DESCRIPTION OF THE INVENTION

Percentages are weight percentages, unless specified otherwise. Percentages of resin weight are on a dry basis, unless specified otherwise. As used herein the term "(meth)acrylic" refers to acrylic or methacrylic. The term "excess liquid" refers to the amount of a liquid phase in a reactor or column that is drained easily via gravity in less than an hour. The term "bed volume" (BV) refers to a volume of liquid equal to the volume of a batch of resin beads in a container, e.g., a reactor or column. The term "styrene polymer" indicates a copolymer polymerized from monomers comprising styrene and/or at least one crosslinker, wherein the combined weight of styrene and crosslinkers is at least 50 weight percent of the total monomer weight. A crosslinker is a monomer containing at least two polymerizable carbon-carbon double bonds, including, e.g., di- and tri-vinyl aromatic or alicyclic compounds; divinyl amides; divinyl ethers of ethylene glycol and diethylene glycol; di-, tri- and tetra-(meth)acrylate esters of ethylene glycol, diethylene glycol, trimethylolpropane, pentaerythritol and dipentaerythritol; and divinyl ether compounds. Preferred crosslinkers include divinylaromatic crosslinkers, e.g., divinylbenzene, and diethylene glycol divinyl ether. In some embodiments of the invention, a styrene polymer is made from a mixture of monomers that is at least 75% styrene and divinylaromatic crosslinkers, more preferably at least 90% styrene and divinylaromatic crosslinkers, and most preferably from a mixture of monomers that consists essentially of styrene and at least one divinylaromatic crosslinker.

In some embodiments of the invention, the polymer is made from monomers that contain from 1 to 10% cross-linking monomers. In some embodiments, the amount of cross-linker is no greater than 8%, alternatively no greater than 7%, alternatively no greater than 6%, alternatively no greater than 5%. In some embodiments, the amount of cross-linker is at least 1.5%, alternatively at least 2%, alternatively at least 2.5%.

The term "acrylic polymer" indicates a copolymer formed from a mixture of vinyl monomers containing at least one (meth)acrylic acid or ester, along with at least one crosslinker, wherein the combined weight of the (meth)acrylic acid(s) or ester(s) and the crosslinker(s) is at least 50 weight percent of the total monomer weight; preferably at least 75%, more preferably at least 90%, and most preferably from a mixture of monomers that consists essentially of at least one (meth)acrylic acid or ester and at least one crosslinker.

The term "gel" or "gellular" resin applies to a resin which was synthesized from a very low porosity (0 to 0.1 cm³/g), small average pore size (0 to 17 Å) and low B.E.T. surface area (0 to 10 m²/g) copolymer. The term "macroreticular" (or MR) resin is applied to a resin which is synthesized from a high mesoporous copolymer with higher surface area than the gel resins. The total porosity of the MR resins is between 0.1 and 0.7 cm³/g, average pore size between 17 and 500 Å and B.E:T. surface area between 10 and 200 m²/g. The term "cation exchange resin" indicates a resin which is capable of exchanging positively charged species with the environment. They comprise negatively charged species which are linked to cations such as Na⁺, K⁺, Ca⁺⁺, Mg⁺⁺, Fe⁺⁺⁺ or H⁺. The most common negatively charged species are carboxylic, sulfonic and phosphonic acid groups. The term "anion exchange resin" indicates a resin which is capable of exchanging negatively charged species with the environment. The term "strong base anion exchange resin" refers to an anion exchange resin that comprises positively charged species which are linked to anions such as Cl⁻, Br⁻, F⁻ and OH⁻. The most common positively charged species are quaternary amines and protonated secondary amines.

The resin of this invention is in the form of beads, powder or fiber. Preferably, the harmonic mean size (diameter) of the resin particles is from 1 µm to 2000 µm, alternatively from 50 µm to 800 µm, alternatively from 100 µm to 800 µm, alternatively from 100 µm to 700 µm, alternatively from 200 µm to 700 µm. The aspect ratio (length/width) of the particles preferably is from 1 to 200.

The term "hydrous oxide" indicates very insoluble compounds in water which are formed from the precipitation of a metal cation with a pH increase in the original solution. The hydrous oxide may be essentially oxides or hydroxides of a single metal or of a mixture of two or more metals. The charge on a hydrous oxide species depends largely upon the degree of acidity of the oxide and the media. They can exist as negatively, neutral or positively charged species. Variations in precipitation conditions for metal ions result in different structures that can be relatively more or less reactive towards various ions in water. The structure of the metallic hydrous oxides can be amorphous or crystalline. The preferred metals are iron, aluminum, lanthanum, titanium, zirconium, zinc and manganese; more preferred are titanium and iron. Fe(III) is an especially preferred metal ion.

An example of the behavior of metal hydroxides at different pH values is that Fe(III) is totally soluble at low pH (less than 1.5) in water at ambient temperature. At high pH and high caustic concentration, another soluble structure is obtained, namely Fe(OH)₄⁻ . The precipitation of Fe(III) starts at a pH of 2-3, depending on the presence of chelating agents and the experimental conditions. The complex stability of Fe(III)Lₓ (L is a ligand) might affect the precipitation pH value. Inside the pH range for precipitation, Fe(III) forms Fe(O)ₓ(OH)_{y} (oxy hydroxides) and/or Fe(OH)₃ (hydroxide). The structure of the precipitated compound among many others might be: Goethite, Akaganeite, Lepidocrocite or Schwertmannite. The temperature at which precipitation occurs also affects the microstructure obtained during the precipitation. Preferably, precipitation is done near ambient temperature, i.e., ca. 20°C to 35°C.

In one embodiment of the invention, the ion exchange resin has at least one substituent selected from hydroxy, ether, amine, quaternary amine, amine oxide and hydroxy amine. In one embodiment of the invention, the resin is a metal-chelating resin which has a chelating substituent selected from phosphonic acids, sulfonic acids, polyethyleneimines, polyamines, hydroxy amines, carboxylic acids, aminocarboxylic acids and aminoalkylphosphonates. Preferred aminocarboxylic substituents include, for example, substituents derived from nitrilotriacetic acid, ethylenediamine tetraacetic acid (EDTA), diethylenetriamine pentaacetic acid, tris(carboxymethyl)amine, iminodiacetic acid, N-(carbamoylmethyl)iminodiacetic acid, N,N-bis(carboxymethyl)-β-alanine and N-(phosphonomethyl)iminodiacetic acid.

Preferably, the level of metal(s) contained in the resin based on the dry weight of the resin is at least 5%, alternatively at least 8%, alternatively at least 12%, alternatively at least 15%, alternatively at least 18%. Preferably the level of metal compound is no more than 45%, alternatively no more than 40%, alternatively no more than 35%, alternatively no more than 30%, alternatively no more than 28%, alternatively no more than 25%. In one embodiment of the invention, the resin is a macroreticular or macroporous resin. In one embodiment of this invention, the base resin for metal loading is an acrylic resin or a styrenic resin, i.e., a resin which is an acrylic polymer or a styrene polymer. In one embodiment of the invention, the resin is an ion exchange resin.

The aqueous solution containing a salt of an amphoteric metal ion may be added to a resin bed contained in a column, or to resin contained in a reactor, in which case preferably the contents are mixed. The aqueous solution can be combined with the resin in one large portion, or in separate portions, with excess liquid drained from the resin beads between portions. Preferably, in the draining steps in the present method, the excess liquid is drained substantially completely, but to facilitate production of resin, the reactor or column may be drained quickly, leaving as much as 30% of the excess liquid behind. In one embodiment of the invention, liquid is allowed to drain for at least 3 hours, alternatively at least 6 hours, alternatively at least 12 hours, alternatively at least 18 hours. In some embodiments of the invention, as much as six or more bed volumes of aqueous solution may be used, and the solution may be added in six or more portions. In one embodiment of the invention, two to three portions of an aqueous solution containing a salt of said amphoteric metal ion are combined with the resin beads, each portion followed by another draining step.

Preferably, the amount of aqueous solution combined with the resin is at least 0.5 bed volumes, alternatively at least 1 BV, alternatively at least 1.5 BV; preferably the amount of aqueous solution is no greater than 5 BV, alternatively no greater than 4 BV, alternatively no greater than 3 BV. Preferably, the concentration of the amphoteric metal ion in the aqueous solution is at least 9%, alternatively at least 10%, alternatively at least 11%; preferably the concentration is no greater than 30%, alternatively no greater than 25%, alternatively no greater than 20%, alternatively no greater than 15%.

In one embodiment, additional portions having a higher concentration of amphoteric metal ion are added and drained, up to six or more total portions. In one embodiment, one, two or three additional portions are added. Preferably, when a higher concentration of amphoteric metal ion is to be added, the concentration of the amphoteric metal ion in the aqueous solution is at least 10%, alternatively at least 12%; preferably the concentration is no greater than 30%, alternatively no greater than 20%, alternatively no greater than 16%.

In one embodiment, when portions of aqueous metal ion are added, the excess liquid is drained until at least 85% of the metal ion added in the previous portion of aqueous metal ion is recovered in the excess liquid drained from the beads, alternatively at least 90%, alternatively at least 95%.

At least 0.3 bed volumes of an aqueous alkali metal hydroxide solution is combined with the drained resin after the metal ion treatment(s) are complete (step (c)). In one embodiment of the invention, at least 0.4 bed volumes are used, alternatively at least 0.5; in this embodiment, no more than 2 bed volumes are used, alternatively no more than 1 bed volume. In one embodiment, the concentration of the alkali metal hydroxide solution is at least 3%, alternatively at least 5%, alternatively at least 7%; in this embodiment the concentration is no greater than 50%, alternatively no greater than 30%, alternatively no greater than 25%, alternatively no greater than 20%, alternatively no greater than 15%. The amount, concentration and rate of addition of the alkali metal hydroxide solution are chosen to raise the pH to greater than 4 within 20 minutes of commencing addition. In one embodiment, the alkali metal hydroxide solution is added so as to raise the pH to greater than 4 within 15 minutes. Preferably, the pH is from 5.5 to 8.5 after addition of the alkali metal hydroxide solution. The amount of alkali metal hydroxide in the alkali metal hydroxide solution preferably is from 0.12 g/g dry resin to 0.75 g/g dry resin, alternatively from 0.37 g/g dry resin to 0.6 g/g dry resin.

Additional aqueous alkali metal hydroxide solution is added in an amount sufficient to maintain liquid-phase pH between 4 and 12 (step (d)). The additional hydroxide is added gradually while monitoring pH in an amount and at a rate sufficient to maintain the pH in the target range. Typically, the amount of hydroxide needed is from 0.1 bed volume of resin to 3 bed volumes of resin. In one embodiment of the invention, after the pH is stable in the target range, an aqueous carbonate or bicarbonate salt is added to the mixture of resin and liquid phase, e.g., aqueous NaHCO₃. Preferably, the amount of carbonate or bicarbonate is from 0.12 g/g dry resin to 0.75 g/g dry resin, alternatively from 0.3 g/g dry resin to 0.6 g/g dry resin. Preferably, the concentration of bicarbonate in the aqueous solution is from 1% to 25%, alternatively from 5% to 10%. In another embodiment of the invention, after adding additional aqueous alkali metal hydroxide solution to maintain liquid-phase pH between 4 and 12, the amount of alkali metal hydroxide introduced into the mixture is further adjusted to maintain a liquid-phase pH between 5 and 8.5.

In one embodiment, the ion exchange resin is an acrylic resin functionalized with the functional group shown below:

RR¹N{(CH₂)ₓN(R²)_{z}(CH₂)_{y}NR³R⁴(R⁵)_{w}

where R denotes the resin, to which the amine nitrogen on the far left is attached via an amide bond with an acrylic carbonyl group or via a C-N bond to a CH₂ group on the acrylic resin; R¹ and R²=H, methyl (Me) or ethyl (Et); x and y =1-4, z = 0-2, w=0-1; and R³, R⁴ and R⁵ = Me, Et, propyl (Pr) or butyl (Bu). A more preferred functionalization would have R attached via an amide bond; R¹=H or Me; z = 0; y = 1-4; w=0-1; R⁵=Me; and R³ and R⁴ = Me or Et. The most preferred embodiment would have R¹=H; y = 3; w=0; and R³ and R⁴ = Me. The amine functional group can be introduced by reacting a diamine which is methylated on one end, e.g., 3-dimethylaminopropylamine (DMAPA) with the acrylic resin at high temperature (120-200°C), under nitrogen pressure between 25-100 psig (138-689 kPa) for 8-48 hours. When R⁵ is present (w=1) the functional group is a quaternary salt, and would have a counter-ion derived from the alkylating agent used to introduce R⁵ or from ion exchange subsequent to alkylation.

In one embodiment, the acrylic resin is a gel constructed from a copolymer of at least one C₁-C₈ alkyl (meth)acrylate, preferably at least one C₁-C₄ alkyl (meth)acrylate, and at least one cross-linker. Preferably, the cross-linker level is from 2% to 10%, more preferably from 2% to 6%. In one embodiment, the copolymer is made from methyl acrylate/divinylbenzene (DVB) with 2-5% DVB and 0-1.0% diethylene glycol divinyl ether as crosslinkers. A more preferred embodiment would have 3-4% DVB and 0.45-0.55% diethylene glycol divinyl ether, with the most preferred being about 3.6% DVB and about 0.5% diethylene glycol divinyl ether. Another embodiment of this invention would use as a base resin for metal loading a macroreticular resin constructed from a copolymer of at least one C₁-C₈ alkyl (meth)acrylate, preferably at least one C₁-C₄ alkyl (meth)acrylate, and at least one cross-linker. Preferably, the cross-linker level is from 6% to 12%. In one embodiment, the copolymer is made from methyl acrylate with 6-9% DVB and 1.1-3.0% diethylene glycol divinyl ether as crosslinkers. A more preferred embodiment would have 7-8% DVB and 1.5-2.5% diethylene glycol divinyl ether, with the most preferred being about 7.6% DVB and about 2.0% diethylene glycol divinyl ether.

In one embodiment of the invention, the resin is a mono-dispersed resin, i.e., one having a uniformity coefficient from 1.0 to 1.3, more preferably from 1.0 to 1.05. The uniformity coefficient is the mesh size of the screen on which about 40% of the resin is retained divided by the mesh size of the screen on which about 90% of the resin is retained. In one embodiment, the mono-dispersed resin is a jetted resin, see, e.g., U.S. Pat. No. 3,922,255. In one embodiment of the invention, the resin is a seed-expanded resin, see, e.g., U.S. Pat. No. 5,147,937.

Plasmapheresis is defined as the process of separating the plasma from blood and manipulating it in some way. For example, in dialysis, the plasma is separated from the blood, passed through the selective cartridge to remove phosphate, then reinfused with the blood and returned to the body. Another method to remove phosphate from the body is to introduce the media inside the body where it will absorb the excess phosphate found in the intestinal tract.

The resins of the present invention are especially suitable for removing phosphates present in aqueous solution at concentrations from 30 to 3000 ppm. In one embodiment of the invention, the concentration of phosphate in the aqueous solution is at least 100 ppm, alternatively at least 200 ppm, alternatively at least 400 ppm, alternatively at least 600 ppm; the concentration of phosphate is no greater than 2600 ppm, alternatively no greater than 2300 ppm. The aqueous solution containing phosphate may be, e.g., gastrointestinal fluid, blood, plasma or dialysis solution. In addition to phosphate and other solutes, the aqueous solution may contain suspended solids. The resin may be ingested orally by a patient suffering from hyperphosphatemia. Preferably the resin is administered in a form comprising an enteric coating. An enteric coating is a barrier applied to oral medication that controls the location in the digestive system where the medicament is made available for absorption or therapeutic action. Enteric refers to the small intestine, therefore enteric coatings prevent release of medication before it reaches the small intestine. Most enteric coatings work by presenting a surface that is stable at acidic pH, but breaks down rapidly at higher pH.

The most currently used enteric coatings are those that remain undissociated in the low-pH environment of the stomach but readily ionize when the pH rises to about 4 or 5. The most effective enteric polymers are polyacids having a pKa of 3-5. Pharmaceutical formulators now prefer to use synthetic polymers to prepare more effective enteric coatings. The most extensively used synthetic polymer is cellulose acetetate phtalate (CAP), which is capable of functioning as an enteric coating. However, a pH greater than 6 is required for its solubility. It is relatively permeable to moisture and gastric fluid and susceptible to hydrolytic decomposition. Another useful polymer is polyvinyl acetate phthalate (PVAP), which is less permeable to moisture and gastric fluid, more stable to hydrolysis, and able to ionize at a lower pH, resulting in earlier release in the duodenum. Other suitable enteric polymers include hydroxypropyl methyl cellulose phthalate (which has properties similar to PVAP), methacrylic acid-methacrylic acid ester copolymers (some of which have a high dissociation constant), cellulose acetate trimellitate (CAT), carboxymethyl ethylcellulose (CMEC) and hydroxypropyl methylcellulose acetate succinate (HPMCAS).

In some embodiments of the invention, the resin is capable of removing phosphate from an aqueous solution in the presence of chloride ion; chloride ion may be present in an amount from 30 ppm to 1500 ppm, alternatively from 100 ppm to 1000 ppm, alternatively from 200 ppm to 700 ppm. In some embodiments of the invention, the resin is capable of removing phosphate from an aqueous solution in the presence of acetate ion; acetate ion may be present in an amount from 30 ppm to 1500 ppm, alternatively from 100 ppm to 1000 ppm, alternatively from 200 ppm to 700 ppm. In some embodiments of the invention, the resin is capable of removing phosphate from an aqueous solution in the presence of lactate ion; lactate ion may be present in an amount from 30 ppm to 1500 ppm, alternatively from 100 ppm to 1000 ppm, alternatively from 200 ppm to 700 ppm. In some embodiments of the invention, the resin is capable of removing phosphate from an aqueous solution in the presence of carbonate ion; carbonate ion may be present in an amount from 30 ppm to 1500 ppm, alternatively from 100 ppm to 1000 ppm, alternatively from 200 ppm to 700 ppm.

Other molecules can be present in the aqueous environment which will not affect the selectivity towards phosphate. Moreover, molecules of relatively high molecular weight, such as proteins will be not accessible to the interior of the resin bead due to their size.

The resin of this invention may be used in contact with an aqueous solution having a pH from 3 to 10, alternatively from 4 to 8, alternatively from 5.5 to 7.5.

The resin of this invention comprises 10% to 50% of an amphoteric metal ion which is present as a hydrous oxide; wherein at least 50% of said metal ion is located in an outer half of the resin bead volume. In one embodiment of the invention, at least 55% of said metal ion is located in the outer half of the resin bead volume, alternatively at least 58%. In one embodiment, at least 25% of the metal ion is located in the outer 20 µm of the bead, i.e., in a shell with a thickness of 20 µm which is located on the outer surface of the bead, alternatively at least 28%.

### EXAMPLES

### Example 1: Iron Loading of an Acrylic Weak Base Gel Ion Exchange Resin

4000 liters of resin (Amberlite^{™} IRA67- weak base acrylic anion exchange resin with 4% crosslinker, and with 3-dimethylaminopropyl (DMAPA) groups attached via an amide linkage) was charged to the reactor. Excess water was drained from the reactor (1 hour). Aqueous ferric sulfate (4000 liters, 40% w/w) was added and the contents agitated for 2 hours. The ferric sulfate solution was drained (1 hour). A second charge of ferric sulfate (4000 liters, 40% w/w) was added and the contents agitated for 2 hours, then drained overnight to achieve at least 90% of recovery of the charged volume of ferric solution. The pH of the ferric solution drained should be between 0.8 - 2.5. 7200 liters of aqueous NaOH solution (8% w/w) was charged in 15 minutes. After completion of the addition, pH of the liquid phase in the reactor was maintained between 4.5 and 10 in the first 40 minutes, between 5 and 8 at 40-80 minutes and between 5.0 and 7.5 at 80-120 minutes. To keep the pH in these ranges, 1125 liters of 8% NaOH were used within 15-80 minutes of this step. The final pH was between 5 and 7.5. The liquid was drained (1 hour), and then 4000 liters of NaHCO₃ (8%) were charged to the reactor as fast as possible, and agitated for 2 hours. The pH was between 7 and 8.2. The liquid was drained from the reactor (45 minutes), and then 6000 liters of water were charged with no agitation. The lot was then agitated for 30 minutes and then the reactor was drained. The resin was washed with excess water to remove particles and clean the resin. The resin contained 15% Fe on a dry basis. The final resin beads had a harmonic mean size of 625 µm.

### Example 2: Iron Loading of an Acrylic Weak Base Gel Ion Exchange Resin

30 g of IRA67 resin were charged to the reactor, and excess water was drained. Aqueous ferric sulfate (12%, 84 mL) was charged to the reactor and agitated for 2 hours, then drained. The ferric sulfate addition cycle was repeated twice more. Aqueous ferric sulfate (13%, 84 mL) was charged to the reactor, agitated for 2 hours, then drained. This second ferric sulfate addition cycle also was repeated twice more. Aqueous NaOH (8%) was added within 2 minutes. The contents were agitated and the pH monitored after the caustic addition; the pH was 6.18 at the end (60 minutes after the NaOH addition). The reactor was drained, and aqueous NaHCO₃ (8%, 84 mL) was added and agitated for 2 hours. The final pH was 6.8. The reactor was drained and the resin washed with 2 liters of water until effluent was clear. This process gave 20% Fe in the resin on a dry basis.

### Example 3: Iron Loading of an Acrylic Weak Base Gel Ion Exchange Resin

357 g of Amberlite^{™} IRA67 resin were charged to the reactor, and excess water was drained. Aqueous ferric sulfate (12% Fe content, 1000 mL) was charged to the reactor and agitated for 2 hours, then siphoned for 8 minutes. 750 ml. of aqueous NaOH (8%) was added for 20 minutes at 37 ml/min. In the first 6.5 minutes, no agitation was used. After 6.5 minutes the agitation was started. The pH at 5.5 minutes was 1.77, and 8.99 at 29 minutes. The final pH was 6.6 at 120 minutes. The solution was siphoned and 500 ml of NaHCO₃ 8% solution was added over 38 minutes. The final pH was 7.4. Excess water was used to wash the material until the effluent was clear. %-Fe in this material was 13.

### Example 4: 74 ml of final resin from Example 1 was charged to the reactor.

140 ml of ferric sulfate solution (40%/w/w) in the reactor mixed 2 hours and the liquid was siphoned out. This last step was repeated 3 times. After siphoning the liquid after the 4^{th} ferric sulfate charge, 120 ml of NaOH 12% were added in one shot to the reactor. After mixing the lot for 3 hours, the lot was washed with 5 liters of water, Buchner dried and packed. This resin had 30 %-Fe dry weight.

### Example 5: 74 ml of an acrylic resin (35% solids) was charged to the reactor.

140 ml of ferric chloride solution (40%-w/v) in the reactor mixed 2 hours and the liquid was siphoned out. This last step was repeated 5 times. After siphoning the liquid after the 6^{th} iron charge, 140 ml of NaOH 12% were added in one shot to the reactor. 40 ml of NaHCO₃ 5 % solution was added to the reactor and after mixing the lot for 12 hours, the lot was washed with 5 liters of water, Buchner dried and packed. This resin had 23 %-Fe dry weight.

### Comparative Example 1: Iron Loading of an Acrylic Weak Base Gel Ion Exchange Resin

4000 liters of resin (Amberlite^{™} IRA67- weak base acrylic anion exchange resin with 3-dimethylaminopropyl (DMAPA) groups attached via an amide linkage) was charged to the reactor. Excess water was drained from the reactor (1 hour). Aqueous ferric sulfate (4000 liters, 40% w/w) was added and the contents agitated for 2 hours. The ferric sulfate solution was drained (1 hour). A second charge of ferric sulfate (4000 liters, 40% w/w) was added and the contents agitated for 2 hours, then drained to achieve at least 90% of recovery of the charged volume of ferric solution. The resin was washed with 80000 liters of water at a flow rate of 8000 liters per hour. The final pH of the effluent was above 2.5. The liquid was drained (1 hour), and then 8000 liters of NaHCO₃ were charged to the reactor as fast as possible, and agitated for 2 hours. The pH was between 6.5 and 7.8. The liquid was drained from the reactor (45 minutes), and then 6000 liters of water were charged with no agitation. The resin was washed with excess water. At the end of the washing step the effluent water from the reactor was clear. The resin contained 5% Fe on a dry basis.

### Comparative Example 2: Iron Loading of an Acrylic Weak Base Gel Ion Exchange Resin

42 ml of resin (Amberlite^{™} IRA67- weak base acrylic anion exchange resin with 3-dimethylaminopropyl (DMAPA) groups attached via an amide linkage) was charged to the reactor. Excess water was drained from the reactor (1 hour). Aqueous ferric sulfate (42 ml, 40% w/w) was added and the contents agitated for 2 hours. The ferric sulfate solution was drained (1 hour). 16 ml of water were charged in 13 minutes. The lot was agitated for 3 minutes and let sit for 30 minutes with no agitation. The liquid was then siphoned for 5 minutes. 42 ml of 10% NaOH solution was added in 31 minutes. The pH was 2.28 after 8 minutes during the addition time. The pH was kept between 3.1-8.99 between 31-64 minutes in the neutralization step. A total of 1.5 BV ( 63 ml) were used in the neutralization step. At the end of 120 minutes the pH was 4.52 and pH 4.17 after 240 minutes. The liquid was siphoned out. 42 ml of a 8% NaHCO₃ solution were charged as fast as possible to the reactor. The lot was agitated for 2 hours, siphoned and washed with excess water. The %-Fe on a dry basis of the resin was 9 %.

### Comparative Example 3:

42 ml of resin (Amberlite^{™} IRA67- weak base acrylic anion exchange resin with 3-dimethylaminopropyl (DMAPA) groups attached via an amide linkage) was charged to the reactor. Excess water was drained from the reactor (1 hour). Aqueous ferric sulfate (42 ml, 40% w/w) was added and the contents agitated for 3 hours. The ferric sulfate solution was drained (1 hour). 800 ml of water were used to wash the resin by plug flow process. The liquid was siphoned for 2 minutes. 84 ml of NaHCO₃ 8% solution were used to neutralize the material. The final pH after the carbonate was 7.5. 800 ml of water were used to wash the resin. The %-Fe on a dry basis of the resin was 10 %.

Resin beads were analyzed by scanning electron microscopy (SEM) and energy dispersive spectroscopy (EDS). The location of iron was determined both by iron/carbon peak ratios (Fe/C) and iron/background peak ratios (Fe/bk.) as a function of outer or inner half of bead volume and distance in microns from the bead surface, and also was predicted as a function of distance based on uniform iron distribution. The results are presented below in Table 1.

**Table 1**

| | Example 1 | | Comp. Example 1 | | |
|---|---|---|---|---|---|
| | %Fe from Fe/C | %Fe from Fe/bk. | %Fe from Fe/C | %Fe from Fe/bk. | predicted |
| outer half | 61% | 61% | 41% | 31% | 50% |
| inner half | 39% | 39% | 59% | 69% | 50% |
| | | | | | |
| 0-20 µm | 32% | 32% | 21% | 13% | 26% |
| 20-40 µm | 22% | 24% | 20% | 19% | 21% |
| 40-60 µm | 15% | 15% | 18% | 19% | 16% |
| 60 µm - center | 30% | 29% | 42% | 49% | 37% |
| | | | | | |
| 0-40 µm | 56% | 55% | 41% | 32% | 46% |
| 40 µm - center | 44% | 45% | 59% | 68% | 54% |

Resin beads were examined by microscopy and determined to contain hydrous iron oxide crystals in the Goethite form with an average length of about 50 nm and an average diameter of about 1 nm.

### Example 6: Equilibrium Test.

Equilibrium testing was done using 0.05 g resin from Example 1, 100 ml of water and 0.02% disodium phosphate and the pH was adjusted with HCl or NaOH at 37°C. The mixture was allowed to react for 2 days. Samples were analyzed using IC (Ion chromatography). The same ion exchange resin was used in our example and in US Patent 6,180,094 B1, Fig. 1.

**Table 6-1. Equilibrium test: 0.05 g of resin, 100 ml of solution, 0.02% disodium phosphate, 37°C.**

| | Media from Example 1 |
|---|---|
| pH | mg PO₄⁻³/g media (%-Fe 15) |
| 4.01 | 225 |
| 5.47 | 223 |
| 5.95 | 225 |
| 6.98 | 229 |
| 7.98 | 229 |

**Table 6-2. Results from US Patent: 6,180,094B1. Equilibrium test: 0.05 g of resin, 100 ml of solution, 0.02% disodium phosphate, 37C. (IRA-67 Results)**

| | Figure 1, 6,180,094 |
|---|---|
| pH | mg PO₄⁻³/g media (%Fe - 1%) |
| 2 | 20 |
| 4 | 100 |
| 6 | 100 |
| 7 | 140 |
| 8.5 | 150 |

### Example 7: Removal of Phosphate from Aqueous Solutions - Column Test

Three columns were packed with 10 mL of the iron-loaded resins of Examples 1, 4, and 5, respectively. 125 ml of aqueous solution containing phosphate 1584 ppm, chloride 4017 ppm and lactate 4045 ppm was recycled through each column until the media's capacity was saturated at a flow rate of 8 mL/min. The removal for each anion is tabulated below. The pH of the initial solution was 5.5, and the temperature was 37°C.

| | resin % solids | %-Fe (dry basis) | PO₄⁻³ Capacity | Final Concentrations | | |
|---|---|---|---|---|---|---|
| | | | mg PO₄⁻³ /mL media | ppm lactate | ppm Cl | ppm PO₄⁻³ |
| Example 1 | 48.5 | 15 | 12.4 | 4043 | 2818 | 600 |
| Example 4 | 55.4 | 30 | 16.3 | 3502 | 2833 | 267 |
| Example 5 | 38.9 | 23 | 12.3 | 3878 | 2809 | 593 |

## Claims

1. A process for removing phosphate ion from an aqueous solution containing phosphate ion; said process comprising steps of:
(a) mixing a resin with at least two bed volumes of an aqueous solution containing a salt of said amphoteric metal ion, and having a metal ion concentration of at least 5%;
(b) draining excess liquid from the resin;
(c) adding at least 0.3 bed volumes of an aqueous alkali metal hydroxide solution having an alkali metal hydroxide concentration of at least 3%, while monitoring pH, at a rate sufficient to raise liquid-phase pH above 4 within 20 minutes;
(d) mixing while adding additional aqueous alkali metal hydroxide solution to maintain liquid-phase pH between 4 and 12;
(e) draining excess liquid from the resin; and
(f) combining the resin with an aqueous solution containing phosphate ion.

2. The process of claim 1 in which the aqueous solution containing phosphate ion contains at least 200 ppm of phosphate ion.

3. The process of claim 2 in which the amphoteric metal ion is Fe(III).

4. The process of claim 3 further comprising adding a bicarbonate or carbonate salt in an amount from 0.12 g/g dry resin to 0.75 g/g dry resin after step (d), and wherein the amount of alkali metal hydroxide is from 0.12 g/g dry resin to 0.75 g/g dry resin, and wherein said at least two bed volumes of an aqueous solution containing a salt of said amphoteric metal ion are added in at least two portions, and excess liquid is drained between portions.

5. The process of claim 4 in which the resin is an ion exchange resin.

6. The process of claim 5 in which the ion exchange resin is an acrylic resin which comprises an amine substituent of structure
RR¹N{(CH₂)ₓN(R₂)}_{z}(CH₂)_{y}NR³R⁴(R⁵)_{w}
where an amine nitrogen bearing substituent R¹ is attached to the resin via an amide bond with an acrylic carbonyl group or via a C-N bond to a CH₂ group on the acrylic gel; R¹ and R²=H, methyl or ethyl; x and y =1-4, z = 0-2, w=0-1; and R3, R⁴ and R⁵ = Me, Et, Pr or Bu.

7. The process of claim 6 in which the amine nitrogen bearing substituent R¹ is attached via an amide bond with an acrylic carbonyl group; R¹ = H; z = 0; y=3; w=0; R³ and R⁴ = methyl; the acrylic resin is an acrylic gel which is a copolymer of at least one C₁-C₈ alkyl (meth)acrylate and 2-10% of at least one cross-linker; and the acrylic gel contains at least 8% Fe(III), based on dry weight of the resin.

8. The process of claim 7 further comprising at least one additional step of mixing the resin with an additional portion of an aqueous solution containing a salt of said amphoteric metal ion and draining excess liquid from the resin.

9. A process for removing phosphate ion from an aqueous solution containing phosphate ion by contacting said aqueous solution with a resin comprising 10% to 50% of an amphoteric metal ion which is present as a hydrous oxide; wherein at least 50% of said metal ion is located in an outer half of a resin bead volume.

10. The process of claim 9 in which the resin is an acrylic ion exchange resin which comprises an amine substituent of structure
RR¹N{(CH₂)ₓN(R²)}_{z}(CH₂)_{y}NR³R⁴(R⁵)_{w}
where an amine nitrogen bearing substituent R¹ is attached to the resin via an amide bond with an acrylic carbonyl group or via a C-N bond to a CH₂ group on the acrylic gel; R¹ and R²=H, methyl or ethyl; x and y =1-4, z = 0-2, w=0-1; and R3, R⁴ and R⁵ = Me, Et, Pr or Bu; and the resin contains 12% to 45% of Fe(III) which is present as a hydrous oxide.
